(19) Europäisches Patentamt
European Patent Office
Office européen des brevets

(11) Veröffentlichungsnummer: **0 140 828 B1**

(12) **EUROPÄISCHE PATENTSCHRIFT**

(45) Veröffentlichungstag der Patentschrift: **27.02.91**

(51) Int. Cl.5: **A61K 47/00**, A61K 9/22

(21) Anmeldenummer: **84810393.3**

(22) Anmeldetag: **09.08.84**

# (54) Vernetzte poröse Polymere für eine kontrollierte Wirkstoffabgabe.

(30) Priorität: **15.08.83 US 523236**

(43) Veröffentlichungstag der Anmeldung:
**08.05.85 Patentblatt 85/19**

(45) Bekanntmachung des Hinweises auf die Patenterteilung:
**27.02.91 Patentblatt 91/09**

(84) Benannte Vertragsstaaten:
**AT BE CH DE FR GB IT LI LU NL SE**

(56) Entgegenhaltungen:
**DE-A- 2 831 274**
**US-A- 4 177 056**
**US-A- 4 192 827**
**US-A- 4 379 864**

**PHARMAZEUTISCHE INDUSTRIE, Band 32, Nr. 6, Juni 1970, Seiten 469-473; H. DETERMANN et al.: "Copolymere von Acrylaten und Acrylamid als Träger fÜr Depot-Präparate"**

(73) Patentinhaber: **CIBA-GEIGY AG**
**Klybeckstrasse 141**
**CH-4002 Basel(CH)**

(72) Erfinder: **Mueller, Karl Friedrich**
**119 West 77th Street**
**New York New York 10024(US)**
Erfinder: **Heiber, Sonia Jaworiw**
**48 David's Lane**
**Bedford Hill New York 10507(US)**

**EP 0 140 828 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der pharmazeutischen Industrie wurde in den letzten Jahren viel Arbeit darauf verwendet, die Wirkung, Sicherheit und Verwendbarkeit von oral verabreichten Wirkstoffen zu verbessern. Diese Erfindung zielt insbesondere darauf ab, die Abgabe eines oral eingenommenen Wirkstoffs über mehrere Stunden zu verlängern. Diese verlängerte Abgabe hat folgende Vorteile: die Spitzenwerte des Wirkstoffspiegels im Blut, die manchmal toxische Spiegel darstellen, werden vermieden, da nicht der gesamte Wirkstoff zur gleichen Zeit in den Magen freigesetzt wird; zweitens werden Wirkstoffkonzentrationen im Blut längere Zeit innerhalb des therapeutischen Bereiches aufrechterhalten, wodurch die Gesamtwirkung des Wirkstoffs erhöht und die für die Behandlung notwendige Gesamtdosis reduziert wird; drittens können Wirkstoffe, die in herkömmlicher Form mehrmals täglich für die Behandlung von chronischen Erkrankungen eingenommen werden müssen, in Dosisformen verabreicht werden, die nur einmal oder zweimal täglich eingenommen werden müssen, was für den Patienten sicherer und zweckmäßiger ist.

Herkömmliche Dosisformen für oral einzunehmende Wirkstoffe sind Tabletten oder Pillen, in denen der Wirkstoff mit einem wasserlöslichen Gummi oder Polysaccharid konfektioniert wird, der bzw. das sich im Magen schnell auflöst oder zerfällt. Eine Erweiterung dieser Technologie sind Tablettenüberzüge und Mehrfachüberzüge um die Tabletten, welche die Zerfall-und Auflösegeschwindigkeit verzögern. Materialien und synthetische Polymere mit verschiedenen Molekulargewichten und Löslichkeiten in Wasser wurden für diesen Zweck ver wendet. In der US-PS 3 432 592 ist eine weiterentwickelte Zusammensetzung dieser Art beschrieben, die aus spritzgegossenen wasserlöslichen Polymeren, wie Polyvinylalkohol, und darin dispergierten Wirkstoffen besteht.

Keines dieser Verfahren ist jedoch zufriedenstellend. Der Tablettenzerfall erfolgt schnell und ist nur schlecht reproduzierbar, da er in großem Ausmaß eine Funktion der physikalischen Bewegung im Magen ist. Deshalb wurden polymere Dosisformen entwickelt, in denen die Wirkstoffabgabe durch Diffusion kontrolliert wird, unabhängig von anderen physikalischen Variablen als die Polymerzusammensetzung und die Struktur. In diesen Dosisformen passiert das Polymere den Körper ohne Abbau.

Beispiele für eine derartige Blockdosisform mit einheitlichen Wirkstoffkonzentrationen sind in der AU-PS 16202 und US-PS 390 050 beschrieben, in denen hydrophile Polymerperlen in Gegenwart eines Wirkstoffs synthetisiert werden. Die US-PS 4 267 138 beschreibt eine orale Dosisform, in der die Freigabe eines Wirkstoffs durch einen Ueberzug kontrolliert wird, der die den Wirkstoff enthaltenen Teilchen umgibt, die in Tabletten komprimiert werden. Die Ueberzüge sind komplizierte Gemische von plastifizierten synthetischen Polymeren und Wasser.

In der US-PS 3 538 214 sind ebenfalls überzogene Tabletten beschrieben, in denen der Überzug als poröse Membran wirkt, die Wasser zur Lösung des eingeschlossenen Wirkstoffs durchläßt, der dann nach außen diffundiert. Poröse Membranen werden dadurch erhalten und ihre Porosität kontrolliert, daß man dem Überzugsmaterial wasserlösliche Hilfssubstanzen zugibt, die vor der Diffusion des Wirkstoffs entfernt werden. Entsprechende von einer Membran umschlossene orale Dosisformen sind in den US-PSen 3 854 480, 3 993 072 und 4 244 941 beschrieben.

Werden die oben angegebenen Membranpräparate in Kombination mit wasserlöslichen Wirkstoffen verwendet, so haben sie alle den Nachteil, daß sie unkontrollbierbar expandieren und wegen des sich aufbauenden osmotischen Druckes zusammenbrechen. Dieses Problem wurde dadurch überwunden, daß man das Prinzip der osmotischen Pumpe anwandte, wie in J. Pharm. Sci. **64** , 1981 (1975) beschrieben. Es besteht aus einer semipermeablen Membran, die nur das Wasser in die Tablette zur Lösung des Wirkstoffs diffundieren läßt, der wiederum durch ein kleines Loch in der Membran hinausgepumpt wird. Obwohl diese Methode mit mäßig löslichen Wirkstoffen gut funktioniert, ist sie auf stark wasserlösliche Bestandteile weniger anwendbar, da der osmotische Druck mit dem sich auflösenden Wirkstoffvorrat schnell zu hoch wird. Wie in allen Membranpräparaten ist die konstante Freigabe nur so lange gewährleistet, als ein Vorrat an nicht aufgelöstem Wirkstoff im Kern vorhanden ist. Ein anderer Nachteil der osmotischen Pumpe ist die hohe Wirkstoffkonzentration, die am Austrittsloch auftritt und zu Reizungen der Magenwand führen kann.

Die oben beschriebenen Block-Polymer-Wirkstoffzusammensetzungen der US-PS 3 390 050 und AU-PS 16202 haben diese Nachteile nicht. In der US-PS 3 390 050 kann keine Endreinigung der Polymere durchgeführt werden, da der Wirkstoff während der Synthese eingearbeitet wird.

Die AU-PS 16202 beschreibt die Verwendung eines in Wasser quellbaren Poly-(2-hydroxyethylmethacrylats) oder von Copolymeren von 2-Hydroxyethylmethacrylat zur Aufnahme von Wirkstoffen aus einer wässrigen lösung. Die getrockneten Polymer-Wirkstoffzusammensetzungen bilden ein oral einnehmbares Präparat mit kontrollierter Abgabe. Ein ähnliches Präparat, jedoch unter Verwendung von in Wasser quellbaren Polymeren (Hydrogelen), die selbst Zweiphasen-Polymere sind und einen viel weiteren Quellbereich in Wasser und organischen Lösungsmitteln aufweisen, ist in den US-PSen 4 192 827 und 4 136 250

beschrieben. Obwohl in all diese Polymere gewisse Wirkstoffe in genügenden Mengen aufgenommen werden können, um die Herstellung von praktischen Dosisformen möglich zu machen, so ist ihr relativ hoher Quellgrad in Wasser (30 bis 80 %) von einem relativ niedrigen Quellgrad in organischen Lösungsmitteln begleitet. Typischerweise liegt das Verhältnis von Quellung (in Prozent) in Ethanol zu Quellung (in Prozent) in Wasser dieser Hydrogele im Bereich von 1:1 zu 2:1.

Da außerdem in diesen als Wirkstoffträger verwendeten Hydrogelen ein hoher Quellgrad in Wasser von einem hohen Quellgrad in organischen Lösungsmitteln begleitet ist, ist entsprechend ein niedriger Quellgrad in Wasser von einem entsprechend niedrigen Quellgrad in organischen Flüssigkeiten, wie Ethanol, begleitet. Diese begrenzte Quellfähigkeit der Hydrogele schränkt die Wirkstoffmenge ein, die von ihnen aus einer wirkstofflösung aufgenommen werden kann, sei sie von wässriger oder organischer Art. Quillt das Polymere in einem geeigneten organischen Lösungsmittel mehr auf als in Wasser, so können durch Beladen aus z.B. ethanolischen Wirkstofflösungen höhere Wirkstoffbeladungen erreicht werden als aus wässrigen Lösungen, vorausgesetzt der Wirkstoff ist in Ethanol löslich. Die Verwendung von organischen Lösungsmitteln zum Beladen von Hydrogelen mit einem Wirkstoff, der später freigesetzt wird, ist in der US-PS 4 192 827 beschrieben worden.

Eine Aufgabe dieser Erfindung ist, eine Zusammensetzung mit kontrollierter Wirkstoffabgabe herzustellen, die eine wirkungsvolle Menge eines pharmazeutischen Medikaments in einem Polymersubstrat enthält, das in polaren organischen Lösungsmitteln in einem viel grösseren Ausmass quellen kann, als herkömmliche, für die Wirkstoffabgabe verwendete Polymere, und das daher mit Wirkstoffen bis zu einer entsprechend höheren Konzentration beladen werden kann, zugleich aber nur einen mässigen Quellgrad in Wasser aufweist.

Die Fähigkeit eines Polymeren, eine große Menge an Wasser zu absorbieren, wurde immer als Notwendigkeit für eine oral verwendbare polymere Matrix zur Wirkstoffabgabe angesehen. Deshalb waren Hydrogele bisher die einzigen Materialien für diesen Zweck. Die Wirkstoffdiffusion durch hydrophobe Polymere ist normalerweise zu langsam, um in einem oralen Wirkstoffabgabepräparat von praktischem Nutzen zu sein. Mit dieser Erklärung stimmt überein, daß hydrophobe Polymer-Medikament-Zusammensetzungen nur als Körperimplantate verwendet wurden, in denen die Wirkstoffabgabe über mehrere Wochen oder sogar Monate erwünscht ist und in denen die Gesamtdosis sehr niedrig ist, wie z.B. bei Steroiden.

Es wurde nun überraschenderweise festgestellt, daß man Polymere in wässriger Suspension oder in der Masse synthetisieren kann, die in organischen Lösungsmitteln, wie Ethanol, zu einem viel größeren Ausmaß quellen können als herkömmliche Hydrogele, wobei sie von 40 bis 90 Gew.-% Ethanol absorbieren können und typischerweise ein Quellverhältnis von Ethanol (in Prozent) zu Wasser (in Prozent) von 3:1 bis 9:1 aufweisen. Deshalb können diese Perlen aus einer Wirkstofflösung in einem organischen Losungsmittel mit einem löslichen Wirkstoff zu entsprechend viel höheren Wirkstoffspiegeln beladen werden, als dies mit herkömmlichen Polymer-Wirkstoffträgern des Hydrogeltyps möglich ist. Zugleich wurde festgestellt, daß selbst dann, wenn die Wassermenge, die von den neuen Polymeren absorbiert wird, niedriger ist als bei herkömmlichen Hydrogelen, nämlich im Bereich von 2 bis 20 Gew.-% Wasser, die neuen Polymere immer noch der Anforderung genügen, aufgenommene Wirkstoffe vollständig innerhalb von einer bis sechs Stunden freizusetzen.

Es ist eine weitere Aufgabe dieser Erfindung, Copolymere herzustellen, die die überraschend hohen Quellgrade in organischen Lösungsmitteln aufweisen und zugleich nur einen mäßigen oder niedrigen Quellgrad in Wasser haben. Deshalb können sie große Mengen an Medikamenten oder anderen Wirkstoffen aus organischen Lösungen aufnehmen, und sind zugleich ideal geeignet für die kontrollierte und verlängerte Freigabe dieser Wirkstoffe in eine wässrige Umgebung, und zwar als orale Dosis in Form von Perlen.

Die Erfindung betrifft somit eine Zusammensetzung mit kontrollierter Wirkstoffabgabe, die aus

(A) einem vernetzten Copolymeren, das in Ethanol zu einem gequollenen, mindestens 40 Gew.-% Ethanol enthaltenden Copolymeren und in Wasser zu einem gequollenen, nicht über 20 Gew.-% Wasser enthaltenden Copolymeren quellen kann, wobei das Quellverhältnis Prozent Ethanol zu Prozent Wasser 2:1 bis 22:1 beträgt und das Copolymere das Copolymerisationsprodukt ist von

(a) 50 bis 99 Gew.-% des Copolymeren (aa) eines wasserunlöslichen monoolefinischen Monomeren oder eines Gemisches solcher Monomeren oder eines Gemisches dieser Monomeren mit 0 bis 45 Gew.-% der Gesamtmonomere (bb) eines wasserlöslichen monoolefinischen Monomeren oder eines Gemisches solcher wasserlöslicher Monomere, worin mindestens ein Drittel des Gewichtes des wasserunlöslichen Monomeren des Bestandteiles (a) ein Monomeres mit einer Alkylgruppe mit 4 bis 21 Kohlenstoffatomen ist, mit

(b) 50 bis 1 Gew.-% des Copolymeren eines Divinyl- oder Polyvinyl-Vernetzungsmittels mit einem Molekulargewicht von 100 bis 10 000, wobei jedoch (b) nicht über 20 Mol-% des Bestandteils (a) ausmacht, und

(B) einer wirkungsvollen Menge eines pharmazeutischen Medikaments besteht.

Vorzugsweise hat das vernetzte Copolymere ein Quellverhältnis von Ethanol (in Prozent) zu Wasser (in Prozent) von 3:1 bis 15:1, insbesondere 4:1 bis 8:1.

Eine bevorzugte Ausführungsform der Erfindung ist eine Zusammensetzung, in der das Copolymere (A) das Copolymerisationsprodukt von 75 bis 98 Gew.-% des Bestandteils (a) und 25 bis 2 Gew.-% des Bestandteils (b) ist.

Eine andere bevorzugte Ausführungsform der Erfindung ist eine Zusammensetzung, in der der Bestandteil (a) 70 bis 95 Gew.-% eines wasserunlöslichen Monomeren (aa) und 30 bis 5 Gew.-% eines wasserlöslichen Monomeren (bb) umfaßt.

Die zur Herstellung der erfindungsgemäßen vernetzten Copolymere verwendeten Vinylmonomere (a) können zweckmäßigerweise in wasserunlösliche (aa) und wasserlösliche (bb) Monomere eingeteilt werden. Die wasserunlöslichen Comonomere umfassen z.B: die Acrylsäure- und Methacrylsäureester und -amide von einwertigen, geradkettigen oder verzweigten Alkoholen mit von 1 bis 20 Kohlenstoffatomen, wobei diese. Alkohole aliphatisch, cycloaliphatisch oder aromatisch sein können. Beispiele dafür sind: Methyl-, Ethyl-, Propyl-, iso-Propyl-, n-, iso-, und tert-Butyl-, Hexyl-, Pentyl-, 2-Ethylhexyl-, n-Octyl-, 1,1,3,3-Tetramethylbutyl-, Decyl-, Tridecyl-, Hexadecyl-, Stearyl-, Cyclohexyl-, Isobornyl-, Dicyclopentadienyl-, Menthyl-, Dicyclopentadienylethyl-; Phenyl-, Benzyl-, Methoxyethyl; Ethoxyethyl-, Furfuryl-, Glycidylacrylat oder -methacrylat sowie die entsprechenden Amide; und Acrylnitril.

Vinylester, wie Vinylacetat, Vinylpropionat oder Vinylbenzoat.

Vinylether, wie Methyt-, Propyl-, Butyl-, Methoxyethyl-vinylether. Fumarat-, Maleat- und Itaconat-Diester der oben angegebenen einwertigen Alkoholreste; Styrol, alpha-Methylstyrol.

Die Monomere können allein oder in Kombination miteinander verwendet werden. Vorzugsweise sind mindestens die Hälfte der wasserunlöslichen Monomere Monomere, die Alkylgruppen mit mindestens vier Kohlenstoffatomen enthalten, wie Butylacrylat oder -methacrylat; 2-Ethylhexylacrylat oder -methacrylat; n-Octylacrylat oder -methacrylat; Di-n-butylfumarat; Benzylmethacrylat; Vinylbutyrat; 1,1,3,3-Tetramethylbutylacrylamid-und -methacrylamid.

Bevorzugte wasserunlösliche Comonomere sind Methylmethacrylat, n-Butylacrylat, n-Butylmethacrylat, 2-Ethylhexylacrylat, n-Octylacrylat und n-Octylmethacrylat, Glycidylmethacrylat und Vinylacetat oder eines ihrer Gemische.

Zusammensetzungen, in denen 10 bis 60 Gew.-% des Bestandteils (a) n-Butylacrylat oder 2-Ethylhexylacrylat sind, sind besonders bevorzugt.

Das wasserlösliche Monomere (bb) ist vorzugsweise Acrylsäure und/oder Methacrylsäure oder eines ihrer wasserlöslichen Derivate, wie die Hydroxyalkylester, in denen die Alkylgruppe 2 bis 4 Kohlenstoffatome hat, z.B. 2-Hydroxyethyl-, 3-Hydroxypropyl-, 2-Hydroxypropyl- oder 2,3-Dihydroxypropylester; sowie ethoxylierte und polyethoxylierte Hydroxyalkylester, wie Ester von Alkoholen der Formel

$HO\text{-}C_mH_m\text{-}O\text{-}(CH_2CH_2O)_n\text{-}H$ in der
$m$ eine Zahl von 2 bis 5 und
$n$ von 1 bis 20 darstellt oder

Ester analoger Alkohole, in denen ein Teil der Ethylenoxid-Einheiten durch Propylenoxid-Einheiten ersetzt ist. Ebenfalls geeignet sind 3-(Dimethylamino)-2-hydroxypropylester und -amide. Eine andere Klasse von geeigneten Derivaten solcher Säuren sind ihre wasserlöslichen Amide, wie nicht substituierte Amide und Amide, die mit niederen Hydroxyalkyl-, niederen Oxaalkyl- oder niederen Dialkylaminoalkylgruppen substituiert sind, in denen die Alkylgruppe 2 bis 4 Kohlenstoffatome hat, wie N-(Hydroxymethyl)-acrylamid und -methacrylamid, N-(3-Hydroxypropyl)-acrylamid, N-(2-Hydroxyethyl)-methacrylamid und N[1,1-Dimethyl-2-(hydroxymethyl)-3-oxabutyl]acrylamid; wasserlösliche Hydrazinderivate, wie Dimethyl-2-hydroxypropylamin-methacrylimid und das entsprechende Derivat der Acrylsäure.

Ebenfalls geeignet, in Kombination mit Comonomeren, sind die niederen Hydroxyalkylmaleinsäureester und Vinylether, in denen die Alkylgruppe 2 bis 4 Kohlenstoffatome hat, z.B. Di-(hydroxyalkyl)-maleate, wie Di-(2-hydroxyethyl)-maleat, und ethoxylierte Hydroxyalkylmaleate, Hydroxyalkylmonomaleate, wie 2-Hydroxyethylmonomaleat, und alkoxylierte Hydroxyalkylmonomaleate, zusammen mit Vinylethern, Vinylestern, Styrol oder im allgemeinen jedem monomeren, das mit Maleaten oder Fumaraten leicht copolymerisiert; Hydroxyalkylvinylether, wie 2-Hydroxyethylvinylether, 4-Hydroxybutylvinylether, zusammen mit Maleaten, Fumaraten oder im allgemeinen allen Monomeren, die leicht mit Vinylethern copolymerisieren.

Andere erfindungsgemäß verwendbare wasserlösliche Comonomere sind:

Alkylether von polyethoxylierten Hydroxyalkylestern der Acryl-und Methacrylsäure, wie Ester von Alkoholen der Formel

$HO\text{-}C_mH_m\text{-}O\text{-}(CH_2CH_2\text{-}O)_n\text{-}CH_3$ in der
$m$ eine Zahl von 2 bis 5 und
$n$ von 4 bis 20 darstellt.

Dialkylaminoalkylester und Amide, wie 2-(Dimethylamino)-ethyl-oder 2-(Diethylamino)-ethylacrylat und -methacrylat, sowie die entsprechenden Amide; Amide, die mit niederen Oxaalkyl-oder niederen Dialkylaminoalkyl-Gruppen substituiert sind, wie N-(1,1-Dimethyl-3-oxabutyl)-acrylamid; wasserlösliche Hydrazinderivate, wie Trialkylamin-methacrylimid, z.B. Triethylamin-methacrylimid und die entsprechenden Acrylsäurederivate. Monoolefinische Sulfonsäuren und ihre Salze, wie Natrium-ethylensulfonat, Natrium-styrolsulfonat und 2-Acrylamido-2-methylpropansulfonsäure; oder monoolefinische Derivate von heterocyclischen, Stickstoff enthaltenden Monomeren, wie N-Vinylpyrrol, N-Vinylsuccinimid, 1-Vinyl-2-pyrrolidon, 1-Vinylimidazol, 1-Vinylindol, 2-Vinylimidazol, 4(5)-Vinylimidazol, 2-Vinyl-1-methylimidazol, 5-Vinylpyrazolin, 3-Methyl-5-isopropenylpyrazol, 5-Methylenhydantoin, 3-Vinyl-2-oxazolidon, 3-Methacrylyl-2-oxazolidon, 3-Methacrylyl-5-methyl-2-oxazolidon, 3-Vinyl-5-methyl-2-oxazolidon, 2- und 4-Vinylpyridin, 5-Vinyl-2-methylpyridin, 2-Vinylpyridin-1-oxid, 3-Isopropenylpyridin, 2- und 4-Vinylpiperidin, 2- und 4-Vinylchinolin, 2,4-Dimethyl-6-vinyls-triazin und 4-Acrylylmorpholin.

Bevorzugt unter den wasserlöslichen Monomeren sind N-Vinyl-2-pyrrolidon, 2-Vinylpyridin, 4-Vinylpyridin, 2-(Dimethylamino)-ethylmethacrylat, N-Methacrylamid, N,N-Dimethylacrylamid, Acrylsäure und Methacrylsäure oder eines ihrer Gemische.

Besonders bevorzugte wasserlösliche Comonomere sind: 2-Hydroxyethylacrylat und -methacrylat, Acryl- und Methacrylsäure; 2-Dimethylaminoethylmethacrylat; N,N-Dimethylacrylamid und N-Vinyl-2-pyrrolidon.

Beispiele für das Divinyl- oder Polyvinyl-Vernetzungsmitel (b) sind die unlöslichen Di- oder Polyacrylste und -methacrylate von Diolen und Polyolen, wie geradkettige oder verzweigte aliphatische Diole, z.B. Ethylenglycol, 1,2-Propylenglycol, 1 ,6-Hexandiol, 1 ,4-Butandiol, 1,4-Butendiol, 1 ,4-Butindiol, Diethylenglycol, Dipropylenglycol, Dipentylenglycol, Polyethylenoxidglycol, Polypropylenoxidglycol, Polytetramethylenoxidglycol, Poly-(ethylenoxid-co-propylenoxid)-glycol, Thiodiethylenglycol, das Reaktionsprodukt eines Diisocyanats (aliphatisch, cycloaliphatisch oder aromatisch) mit der doppelten äquivalenten Menge an Hydroxyalkylacrylaten oder -methacrylaten; die Reaktionsprodukte von Vorpolymeren mit endständigen Isocyanatgruppen, die sich von Polyesterdiolen. Polyetherdiolen oder Polysiloxandiolen ableiten, wie sie in der Polyurethantechnologie bekannt sind; mit einem Molekulargewicht von 500 bis 10 000, mit dem doppelten Äquivalentgewicht von Hydroxyalkylmethacrylaten. Andere Di- und Polyvinyl-Vernetzungsmittel, einschließlich Divinylether und Diallylverbindungen, sind in der US-PS 4 112 827, geeignete Polysiloxan-di- und -polyvinylverbindungen in der US-PS 4 136 250 beschriehen.

Beispiele von geeigneten Vernetzungsmitteln sind: Trimethylolpropantriacrylat, Neoentylglycol-diacrylat, Pentaerythritund Dipentaerythrit-di-, -tri-, -tetra-, -penta-, -hexa-acrylate; Ethylenglycol- und Diethylenglycol-acrylate, Divinylether; Divinylbenzol, Allylmethacrylat; Diallylmaleat, Diallylamin; Divinylsulfon; Triallylcyanurat.

So ein Vernetzungsmittel ist ein hydrophobes Makromeres mit zwei endständigen Olefinresten mit einem Molekulargewicht von etwa 400 bis etwa 8000 der Formel

$$\overset{R_3}{\overset{|}{HC}} = \overset{R_2}{\overset{|}{C}} - X - Y - R_1 - Y - X - \overset{R_2}{\overset{|}{C}} = \overset{R_3}{\overset{|}{CH}}$$

in der $R_1$ eine polykondensierte Kette mit einem Molekulargewicht von etwa 200 bis 8000 und der Rest eines Poly-(propylenoxid)-oder Poly-(tetramethylenoxid)-glycols mit Etherbindungen ist,

$R_2$ ein Wasserstoffatom, eine Methylgruppe oder - $CH_2COOR_4$ ist, in der $R_4$ ein Wasserstoffatom oder eine Alkylgruppe mit bis zu 10 Kohlenstoffatomen ist,

$R_3$ ein Wasserstoffatom oder $COOR_4$ ist, mit der Maßgabe, daß mindestens einer der Reste $R_2$ und $R_3$ ein Wasserstoffatom ist,

X die Oxagruppe, -COO- oder - $CONR_5$- ist, wobei $R_5$ ein Wasserstoffatom oder eine Alkylgruppe mit bis zu 5 Kohlenstoffatomen ist und

Y eine direkte Bindung oder der Rest - $R_6$-$Z_1$-CO-NH-$R_7$-NH-CO-$Z_2$-ist, wobei $R_6$ an X gebunden ist und einen verzweigten oder geradkettigen Alkylenrest mit bis zu 7 Kohlenstoffatomen darstellt, $Z_1$ und $Z_2$ die Oxagruppe oder $NR_5$ sind und $R_7$ ein zweiwertiger aliphatischer oder aromatischer Diisocyanatrest ist, mit der Maßgabe, daß wenn X die Oxagruppe ist, Y keine direkte Bindung ist und $R_2$ und $R_3$ Wasserstoffatome sind.

$R_1$ ist vorzugsweise eine Poly-(propylenoxid)- oder Poly-(tetramethylenoxid)-Kette mit einem Molekulargewicht von etwa 600 bis etwa 4000. Das Makromere ist vorzugsweise ein Polytetramethylenoxid-gylcol mit

einem Molekulargewicht von etwa 600 bis etwa 4000, das Toluol- oder Isophorondiisocyanat-Endgruppen aufweist und mit 2 Mol eines Hydroxyalkylacrylats oder -methacrylats umgesetzt wird, wobei der Alkylrest 2 bis 4 Kohlenstoffatome hat.

Andere bevorzugte Vernetzungsmittel umfassen $C_{2-6}$-Alkylendiacrylate oder -methacrylate, 3-Oxapentamethylen-diacrylat, 3-Oxapentamethylen-dimethacrylat und Trimethylolpropan-triacrylat sowie eines ihrer Gemische.

Die Menge an Vernetzungsmittel kann von 1 bis 50 Gew.-% variieren; sie sollte jedoch nicht über 20 Mol-% des Gesamtmonomerengemisches ausmachen. Bevorzugte Vernetzungsmittel sind solche mit einem Molekulargewicht von 500 bis 5000, die Polyalkylenether-Einheiten enthalten, insbesondere die in der US-PS 4 192 827 beschriebenen Verbindungen.

Das während der Polymerisation vorhandene inerte Verdünnungsmittel kann jede organische Flüssigkeit sein, die das Monomerengemisch klar löst. Es muß jedoch nicht ein Lösungsmittel für das Polymere sein und kann in seiner Lösungsfähigkeit von einem thermodynamisch guten Lösungsmittel für Monomere und Polymere bis zu einem thermodynamisch schlechtem Lösunsmittel für das Polymere variieren.

Inerte Verdünnungsmittel, die thermodynamisch gute Lösungsmittel für die Monomere, jedoch schlechte Lösungsmittel für die Copolymere sind, werden üblicherweise bei der Herstellung von makroporösen Ionenaustauscherharzen verwendet, die Copolymere von Styrol und Divinylbenzol sind. Die Herstellung von makroporösen Hydrogelen nach dem gleichen Prinzip ist in DD-PS 66283 und US-PS 4 184 020 beschrieben. In allen Fällen war die Aufgabe, makroporöse Hydrogele für die Gelchromatographie oder Gaschromatographie herzustellen; keines wurde für orale Zusammensetzungen mit kontrollierter Wirkstoffabgabe verwendet. Das ist verständlich, da die Abgabegeschwindigkeit wegen ihrer starken hydrophilen Eigenschaften unzweckmässig hoch wäre.

Die Verwendung von inerten Verdünnungsmitteln, die thermodynamisch gute Lösungsmittel für sowohl die Monomere als auch die entstehenden Copolymere sind, gibt mikroporöse Polymere mit größeren Abständen zwischen den Vernetzungen (expandierte Gelstruktur), die mit der mikroporösen, homogenen Struktur der in Abwesenheit solcher Verdünnungsmittel hergestellten Polymeren vergleichbar sind.

Eine bevorzugte Ausführungsform der Erfindung betrifft Zusammensetzungen, die unter Verwendung von inerten Verdünnungsmitteln hergestellt werden, die gute Lösungsmittel für die Monomere sind und deren Lösungsfähigkeit für die Copolymere von gut bis schlecht variiert. Ein derartiges Copolymeres mit einem bestimmten Bereich von mikroporösen zu makroporösen Strukturen kann durch geeignete Wahl des verwendeten Verdünnungsmittels hergestellt werden. Die gewünschte Struktur hängt wiederum von den Löslichkeitsegenschaften des aufzubringenden Wirkstoffs wie auch von seiner Dosis ab.

Ist das Verdünnungsmittel ein gutes Lösungsmittel für das Polymere, so erhält man ein Produkt mit einer mikroretikulären, d.h. mikroporösen, Netzstruktur. Diese Struktur ist mit der vergleichbar, die man in Abwesenheit von Lösungsmitteln erhält, wenn die Polymerisation in der Masse durchgeführt wird, unterscheidet sich von ihr jedoch durch die ausgedehntere und offenere Netzstruktur, was auf die Anwesenheit des guten Lösungsmittels zurückzuführen ist. Ist andererseits das Verdünnungsmittel nur ein Lösungsmittel für das Monomere, jedoch kein Lösungsmittel oder ein Fällungsmittel für das Polymere, so erhält man ein Produkt mit einer makroporösen oder makroretikulären Struktur, die zweiphasig ist (Gas/ Polymeres). Obwohl die Extreme der beiden Möglichkeiten gut voneinander unterschieden werden können, bilden die Zwischenstufen von mikro- und makroretikulären Polymeren einen fließenden Übergang.

Die Lösungsfähigkeit und die Löslichkeitsparameter des Verdünnungsmittels sind im allgemeinen eine Funktion seiner chemischen Natur, wie des Grades der Wasserstoffbindungen, der polaren und nicht polaren Wechselwirkungen, der Anwesenheit von Heteroatomen und im allgemeinen des Ähnlichkeitsgrades des Verdünnungsmittels zu dem verwendeten Monomeren. Die Wirkung der Phasentrennung während der Polymerisation in Anwesenheit eines inerten Verdünnungsmittels wird durch eine Erhöhung der Vernetzungsdichte noch gesteigert. Die Wahl der richtigen Verdünnungsmittel und der richtigen Menge an Vernetzungsmittel, um eine mikro- oder makroretikuläre Struktur zu erhalten, ist eine leichte Aufgabe für den Fachmann auf dem Gebiet der Polymerchemie und insbesondere auf den Gebiet der Herstellung von Ionenaustauscherharzen.

Eine andere Gruppe von Verdünnungsmitteln, die sehr schlechte Lösungsmittel für die Polymere sind, sind andere Polymere. Es ist wohlbekannt, dass sich zwei verschiedene Polymere im wesentlichen nicht ineinander lösen, was auf ihrer extrem niedrigen Vermischungswärme beruht. Während sich polymere Verdünnungsmittel in dem Monomerengemisch lösen können, tritt während der Polymerisation Phasentrennung zwischen den beiden Polymeren auf. Nach der Extraktion des inerten polymeren Verdünnungsmittels erhält man ein Produkt mit einer ausgedehnten Netzstruktur, die wahrscheinlich eher makroretikulär als mikroretikulär ist. Derartige polymere Verdünnungsmittel, wie Polyalkelenther-glycoleoder Polyester sind deshalb erfindungsgemäss besonders bevorzugt.

Geeignete Verdünnungsmittel mit niedrigem Molekulargewicht sind: Ester, wie Ethylacetat, Butylcellosolveacetat, Butylacetat, Isobutylacetat, Methylcellosolveacetate; Ether, wie Methylphenylether, Tetrahydrofuran; Alkohole, wie Ethanol, Isopropanol, n-, iso- und tert-Butanol, Laurylalkohol, Octanol, Decanol, Dodecanol, Butylcellosolve, Ethylcellosolve, Butylalkohol, Cyclohexanol; Ketone, wie Methylethylketon, Methyl-isobutylketon; Amide, wie Dimethylformamid, Formamid, Acetamid, Dimethylacetamid; Dimethylsulfoxid; Sulfolan; N-Methyl-2-pyrrolidon. Ebenfalls geeignet sind Kohlenwasserstoffe, wie Hexan, Heptan, Cyclohexan oder halogenierte Kohlenwasserstoffe, wie Tetrachlorethylen, Trichlorethan oder Trichlorethylen.

Bevorzugte inerte Verdünnungsmittel sind Polyalkylenetherglycole, wie Polyethylenoxid-glycol, Polypropylenoxid-glycol und Poly-n-butylenoxid-glycol, und ihre Blockcopolymere, Octanol, Decanol, Dodecanol, Heptan oder Isobutylacetat.

Jeder zur Behandlung des Körpers verwendete Wirkstoff, sowohl lokal als auch systemisch, kann als Wirkstoff in die erfindungsgemäßen copolymeren Träger einverleibt werden. "Wirkstoff" wird hier in seinem breitesten Sinne verwendet und umfaßt jede Zusammensetzung, die eine pharmakologische oder biologische Reaktion hervorruft.

Erfindungsgemäße, für die Therapie geeignete Wirkstoffe umfassen ohne ßegrenzung auch die Wirkstoffe, die in der US-PS 3 732 865 angegeben sind.

Andere Wirkstoffe mit der gleichen oder einer anderen physiologischen Aktivität als die der oben beschriebenen Wirkstoffe, können ebenfalls in den erfindungsgemässen Trägern verwendet werden. Geeignete Wirkstoffgemische können natürlich mit der gleichen Leichtigkeit wie bei Einzelkomponentensystemen eingebracht werden.

Die Wirkstoffe können in verschiedenen Formen vorliegen, wie als ungeladene Moleküle, Bestandteile von Molekülkomplexen oder nicht reizende pharmakologisch verträgliche Salze, z.B. das Hydrochlorid, Hydrobromid, Sulphat, Phosphat, Nitrat, Borat, Acetat, Maleat, Tartrat, Salicylat usw. Für saure Wirkstoffe können die Salze von Metallen oder Aminen oder organische Kationen (z.B. quatäre Ammoniumionen) verwendet werden. Ausserdem können einfache Derivate der Wirkstoffe (wie Ether, Ester oder Amide), welche die gewünschten Retentions- und Abgabeeigenschaften haben und durch den pH-Wert, Enzyme usw. des Körpers leicht hydrolisiert werden, verwendet werden.

Die Menge an in den Träger einverleibtem Wirkstoff hängt stark von dem bestimmten Wirkstoff, der gewünschten therapeutischen Wirkung und der Zeit ab, die notwendig ist, bis der Wirkstoff freigesetzt wird. Da eine Vielzahl von Trägern in verschiedenen Größen und Formen vollstandige Dosissysteme für die Therapie einer Vielzahl von Krankheiten zur Verfügung stellen) sollen, gibt es keine kritische obere Grenze für die in den Träger einzuverleibende Menge an Wirkstoff. Auch die untere Grenze hängt von der Wirkung des Wirkstoffes und seiner Freigabedauer vom Träger ab. Deshalb ist es nicht praktisch, einen Bereich für die therapeutisch wirkungsvolle Menge eines vom Träger freizusetzenden Wirkstoffs anzugeben.

Bevorzugte, gemäß der Erfindung einzuverleibende Wirkstoffe sind solche, die für eine Langzeitbehandlung bestimmt sind, so daß tägliche Mehrfachdosen vermieden werden können. Beispiele sind Anabolika, wie Methandrostenolon, Analgetika, wie Acetylsalicylsäure, Phenylbutazon oder Methadon, Androgene, wie Methyltestosteron, Antibiotika, wie Rifampin, Antidepressiva, wie Imipramin oder Maprotilin, Antidiabetika, wie Phenformin, Anticonvulsiva, wie Carbamazepin, Antihistaminika, wie Tripelenamin, Antihypertensiva, wie Hydralazin, infektionshemmende Mittel, wie Trimethoprim, Antiparasitika, wie Nifurimox, Antiparkinson-Mittel, wie Levodopa, Antiphlogistika, wie Naproxen, Antitussiva, wie Benzonstat, Appetit unterdrückende Mittel, wie Mazindol, Bronchodilatoren, wie Fenoterol, Coronardilatoren, wie Fenalcomin, Corticoide, wie Dexamethason, Cytostatika, wie Floxuridin, Diuretika, wie Hydrochlorthiazid, Hypnotika, wie Glutethimid, Neuroleptika, wie Reserpin oder Thioridazin, Psyhchoanaleptika, wie Methylpenidat, Beruhigungsmittel, wie Diazepan, Gichtmittel, wie Sulfinpyrazon, Vasodilatoren, wie Isoproterenol.

Unter den am meisten bevorzugten Wirkstoffen sind Oxprenolol• HCl (TRASICOR$^R$), Diclofenac-Natrium (VOLTAREN$^R$), Baclofen (LIORESAL$^R$), Metropolol•HCl (LOPRESSOR$^R$), Betablocker, wie Oxprenolol und Propanolol, Calcium-Kanalolocker, wie Nifedipin$^R$ und Verapamil$^R$.

Die erfindungsgemäßen Copolymere werden durch mit freien Radikalen initiierte Polymerisation synthetisiert unter Verwendung von entweder Redoxkatalysatoren, Peroxyverbindungen oder Azoverbindungen; typische Initiatoren sind Lauroylperoxid, tert.-Butylperoctoat und Azo-biiso-butyronitril. Wie dem Fachmann geläufig, sind verschiedene Peroxy- und Azoverbindungen im Handel erhältlich, die verwendet werden können. Die Polymerisation mit freien Radikalen kann mit UV initiiert werden in Gegenwart von herkömmlicherweise verwendeten UV-Initiatoren und Sensibilisatoren, wie Benzophenon, Benzoin, Diethoxyacetophenon (DEAP) und verwandten Verbindungen. Bestrahlung mit Elektronenstrahlen kann auch verwendet werden, wenn das Polymere in Form von Filmen, Überzügen oder Folien hergestellt wird.

Die Polymere werden in Form von Perlen durch Suspensionspolymerisation in einem wässrigen Medium hergestellt. Um die Löslichkeit der wasserlöslichen vorhandenden Comonomeren zu vermindern,

ist das Medium vorzugsweise eine Lösung eines anorganischen Salzes, im allgemeinen konzentrierte Natriumchloridlösung. Als Suspensionsmittel können entweder polymere Suspensionsmittel verwendet werden, wie Poly-(vinylpyrrolidon), Poly-(vinylalkohol) oder Poly-(hydroxyethylcellulose), oder anorganische unlösliche Salze, wie Calciumphosphat, Calciumoxalat, Calciumcarbonat, Calciumsulfat oder Magnesiumphosphat, oder unlösliche Hyroxide, wie $Mg(OH)_2$, $Al(OH)_3$ oder $Ti(OH)_4$. Ein Verfahren, das zur Herstellung der erfindungsgemäßen Polymere in Form von Perlen verwendet werden kann, ist in der US-PS 4 224 427 beschrieben. Nach der Synthese werden die Polymerperlen gründlich mit Ethanol oder Gemischen von Ethanol/Wasser oder anderen geeigneten Lösungsmitteln extrahiert und bis zur Gewichtskonstanz getrocknet. Die erfindungsgemäßen Polymere können auch durch Massenpolymerisationsverfahren in Form von Folien, Filmen oder Formen hergestellt werden.

Die erfindungsgemässen Copolymere können durch Giessen und durch thermisch oder durch UV-Bestrahlung initiierte Polymerisation zu Formkörpern, Stäben, Filmen oder Folien und Ueberzugen verarbeitet werden.

Nach der Synthese sind die Polymere durch ihren Quellgrad in Wasser und Ethanol, ihre Fähigkeit, einen Wirkstoff aufzunehmen und diesen Bestandteil in eine wässrige Umgebung abzugeben, charakterisiert; dies wird eingehender in den Beispielen beschrieben.

Nach dem Trocknen werden die Polymerperlen mit einem bestimmten aktiven Bestandteil, wie einem Wirkstoff, getränkt. Als Lösungsmittel für den Wirkstoff kann jedes Lösungsmittel verwendet werden, das (a) den Wirkstoff löst, (b) das Polymere quillt und (c) quantitativ entfernt werden kann. Es werden vorzugsweise niedrig siedende Lösungsmittel, wie Methanol oder Ethanol, Methylenchlorid, Aceton oder Gemische dieser Lösungsmittel verwendet. Wässrige alkoholische Lösungen sind ebenfalls geeignet. Die beste Wahl der Lösungsmittel hängt von der Löslichkeit des Wirkstoffes und dem gewünschten Beladungsgrad ab. Im allgemeinen jedoch ist Ethanol, wie aus den Beispielen ersichtlich ist, für viele wasserlösliche Wirkstoffe auch ein gutes Lösungsmittel, und die hohe Ethanolquellfähigkeit der erfindungsgemäßen Polymere macht eine hohe Beladung mit Wirkstoff aus Ethanollösungen möglich und ist deshalb bevorzugt.

Eine besonders hohe Beladung mit einigen Wirkstoffen erhält man, wenn die Polymersynthese in Gegenwart eines inerten Verdünnungsmittels durchgeführt wird.

Zur Beladung des Copolymeren mit einem aktiven Bestandteil wird das Copolymere in einer Lösung des Wirkstoffs in Ethanol, Methanol oder einem anderen organischen Lösungsmittel oder einem Gemisch solcher Lösungsmittel, das auch Wasser enthalten kann, ins Gleichgewicht gebracht. Dann wird die beladene Zusammensetzung entfernt, z.B. durch Filtrieren; das Lösungsmittel wird aus der Zusammensetzung abgezogen, und zwar durch Trocknen in der Wärme und/oder im Vakuum.

Es wurde ausserdem festgestellt, dass eine besonders hohe Wirkstoffbeladung dadurch erreicht werden kann, daß man die Polymerisation in Gegenwart eines inerten Verdünnungsmittels durchführt, was zu einem Endpolymeren mit erhöhter Porengröße führt und das deshalb noch größere Mengen an Lösungsmittel und Wirkstoff absorbieren kann.

Mit Wirkstoff beladene Zusammensetzungen in denen das Polymere in Gegenwart solcher inerter Verdünnungsmittel, wie Poly-(propylenglycol), Decanol oder Dodecanol erhalten worden ist, sind deshalb eine bevorzugte Ausführungsform der Erfindung.

Trotz der hohen Wirkstoffbeladungen haben auf diese Weise behandelte Perlen eine verlängerte und verzögerte Wirkstoffabgabe.

Das Beladen einer Polymerperle mit genügend Wirkstoff, um eine praktische Dosisgröße herzustellen, ist nur eine der Anforderungen für eine erfolgreiche orale Dosisform; die Abgabe des Wirkstoffs innerhalb einer zweckmäßigen Zeit ist eine andere Anforderung. In vielen Fällen ist es ein genügend großer Vorteil gegenüber herkömmlichen Dosisformen, den Wirkstoff aus einer polymeren Blockmatrix freizusetzen, da ein solches System die Möglichkeit des Wirkstoffmißbrauchs vermindert. Obwohl die Freigabe nicht konstant sondern konzentrationsabhängig ist, so ist sie doch genügend verlangsamt, um mögliche toxische Überdosiereffekte zu verhindern.

In Fällen, in denen eine konstantere Freigabe erwünscht ist, wurde überraschenderweise festgestellt, daß die erfindungsgemäßen Polymer-Wirkstoff-Zusammensetzungen besonders geeignet sind zur Durchführung eines Verfahrens der "kontrollierten Extraktion".

Bei diesem Verfahren wird ein dünner Oberflächenbereich der wirkstoffbeladenen Perle durch Extraktion während einer bestimmten Zeit mit einem niedrig siedenden Lösungsmittel, wie Aceton, wirkstoffrei gemacht und dann schnell getrocknet. Warum diese Polymere dieses unerwartet günstige Verhalten zeigen, ist nicht ganz klar; es könnte sich aber um eine Phaseninversion der Polymersegmente auf der Perlenoberfläche unter dem Einfluß eines Lösungsmittels handeln.

Es wurde außerdem überraschenderweise festgestellt, daß die Verwendung von polymeren inerten Verdünnungsmitteln während der Synthese der erfindungsgemäßen Polymerperlen nach der Extraktion

Perlen mit einer spezifisch modifizierten Netzstruktur gibt, die unerwartet gute Wirkstoffabgabeeigenschaften haben, insbesondere verhindern sie eine übermäßige Schwanzbildung bzw. ein übermäßiges Verschleppen.

Als Formkörper, z.B. als Stäbe, sind die erfindungsgemässen Polymere auch als Wirkstoffträger für Implantate geeignet, da ihre Blut- und Gewebeverträglichkeit ausgezeichnet ist.

In Form von Filmen oder Folien sind die erfindungsgemäßen Polymere als Wirkstoffträger für transdermale Präparate oder Wundbehandlung und Verbände geeignet.

In den folgenden Beispielen werden die Quellgrade (DS) in Wasser oder Ethanol in Prozent Wasser oder Ethanol in dem gequollenen Polymeren angegeben, nämlich:

$$\text{DS in Wasser oder Ethanol (\%)} = \frac{\text{Gewicht des gequollenen Polymeren} - \text{Gewicht des trockenen Polymeren}}{\text{Gewicht des gequollenen Polymeren}} \times 100$$

Die Wirkstoffkonzentrationen (DC) der mit Wirkstoff beladenen Polymeren werden entsprechend in % des mit Wirkstoff beladenen Polymeren angegeben.

Das Quellverhältnis der Polymeren wird angegeben als Verhältnis von Prozent Ethanol zu Prozent Wasser.

In den folgenden Beispielen bezieht sich "MAC" auf ein difunktionelles Vernetzungsmittel, das man dadurch erhält, daß man 2 Mol Isophoron-diisocyanat mit 1 Mol Poly-n-butylenoxid-diol mit einem mittleren Molekulargewicht von 2006 umsetzt, und dann mit 2 Mol 2-Hydroxyethylmethacrylat an den Enden propft, wie es in Beispiel 1 beschrieben ist.

Die folgenden Abkürzungen werden verwendet:

HEMA : 2-Hydroxyethyl-methacrylat
NVP : N-Vinyl-2-pyrrolidon
MMA : Methyl-methacrylat
EHA : 2-Ethylhexylacrylat
BA : Butylacrylat
t-OCT : tert.-Octylmethacrylamid (= 1,1,3,3-Tetramethylbutyl-methacrylamid)

DMA : Dimethylacrylamid
MAC : Macromeres (B) wie in Beispiel 1 hergestellt
GMA : Glycidylmethacrylat

Beispiel 1: Herstellung von Polymerperlen durch Suspensions polymerisation

Ein 1 000 ml-Kunststoffkolben mit glatten Wänden wird ausgerüstet mit einem Rückflußkühler, einem Stickstoff-Einlaßstutzen, einem mit einem Temperaturregler verbundenen Thermometer, einem Leitblech und einem Rührer des Ankertyps, der von einem Motor mit variabler Geschwindigkeit angetrieben wird. Ein langsamer Stickstoffstrom wird während der ganzen Zeit durch den Reaktionskolben geleitet.

In den Kolben werden 360 g einer 20-proz. (Gewichtsbasis) wässrigen Natriumchloridlösung, dann 12,5 g (0,0615 Mol) Magnesiumchlorid-hexahydrat eingespeist. Die Lösung wird unter schnellem Rühren langsam auf 80 °C erhitzt. Diese Lösung wird dann tropfenweise mit 123 ml (0,123 Mol) einer 1 n Natriumhydroxidlösung versetzt; es bildet sich ein feiner gelatinöser Magnesiumhydroxid-Niederschlag im Reaktionskolben. Nach der Zugabe des gesamten Natriumhydroxids wird die Rührgeschwindigkit auf 100 Upm

vermindert; ein Gemisch von 42 g 2-Hydroxyethylmethacrylat, 110 g Methylmethacrylat und 24 g N-Vinylpyrrolidon und 24 g Macromeres (b), das gelöst 0,2 g tert.-Butylperoctoat als freien Radikal-Polymerisationsinitiator enthält, wird zugegeben. [Das Macromere (b) wird dadurch hergestellt, dass man 60 g (etwa 0,024 Mol) eines Poly-(tetramethylenoxid-glycols) mit einem mittleren Molekulargewicht von 2000, das Isophoron-diisocyanat-Endgruppen (= 3-Isocyanat-methyl-3,5,5-trimethylcyclohexylisocyanat) aufweist, in 40 g (0,31 Mol) 2-Hydroxyethylmethacrylat (HEMA) löst und das Gemisch während 72 h bei Raumtemperatur stehenlässt. Am Ende dieser Zeit wird das Verschwinden der endstandigen Isocyanatgruppen durch Verschwinden der charakteristischen Infrarot-Spektralbande bei 2270 $cm^{-1}$, die mit der -NCO-Gruppe assoziiert ist, bestätigt].

Uas Reaktionsgemisch wird unter Stickstoff bei 100 Upm und 75 °C 3 h gerührt. Die Temperatur wird dann während 1 h auf 100 °C angehoben; dann wird der Kolben auf Raumtemperatur abgekühlt. 10 ml konzentrierter Salzsäure werden zugegeben, um das Magnesiumhydroxid-Suspensionsmittel aufzulösen. Das Reaktionsgemisch wird durch feines Käseleinen filtriert. Die isolierten Produktperlen werden mit 2000 ml Wasser gewaschen und über Nacht in 500 ml Ethanol eingeweicht, um eventuell zurückgebliebenes Monomeres zu extrahieren. Die Perlen werden dann dadurch isoliert, daß sie durch einen Polyestertuch-Beutel filtriert werden, der dann zugenäht wird und in einem Haushaltswäschetrockner getrocknet wird. Man erhält einheitliche kugelförmige Perlen in einer Ausbeute von 184 g (92,7 %) mit einem mittleren Durchmesser von 0,98 mm und einem Quellvermögen in Wasser von 16 % und in Ethanol von 38 %.

Die folgenden Beispiele beweisen die einzigartigen Quelleigenschaften der erfindungsgemäßen Polymere (großes Verhältnis von Prozent Ethanol zu Prozent Wasser; Prozent $H_2O$ > 1).

Beispiele 2 bis 12:

Gemäß der Arbeitsweise des Beispiels 1 werden Perlen der folgenoen Zusammensetzungen und physikalischen Eigenschaften hergestellt.

Zusammensetzungen (Gew.-%)

| Beispiel | Comonomere A hydrophobe ($A_1$) | | | | Comonomere A hydrophile ($A_2$) | | | Vernetzungsmittel (B) | Perlengröße (mm) | % Quellung in Ethanol | % Quellung in Wasser | Quellverhältnis (% Ethanol/ % Wasser) |
|---|---|---|---|---|---|---|---|---|---|---|---|---|
| | MMA | BA | EHA | t-OCT | HEMA | NVP | DMA | MAC % | | | | |
| 1 | 55 | - | - | - | 21 | 12 | - | 12 | 0.98 | 38 | 14 | 2.7 |
| 2 | 27.5 | - | 27.5 | - | 21 | 12 | - | 12 | 1.05 | 60 | 11 | 5.5 |
| 3 | 27.5 | - | 27.5 | - | 21 | - | 12 | 12 | 0.96 | 63 | 12 | 5.2 |
| 4 | 27.5 | 27.5 | - | - | 21 | 12 | - | 12 | 1.07 | 58 | 10 | 5.8 |
| 5 | 27.5 | - | - | 27.5 | 21 | - | 12 | 12 | 0.85 | 70 | 9 | 7.8 |
| 6 | 15 | - | 40 | - | 21 | 12 | - | 12 | 1.20 | 50 | 11 | 4.5 |
| 7 | 40 | - | 13 | - | 21 | 12 | - | 12 | 1.04 | 64 | 12 | 5.3 |
| 8 | 35 | - | 35 | - | 8 | - | 10 | 12 | 1.05 | 58 | 3 | 19.9 |
| 9 | 18 | 50 | - | - | 8 | - | 12 | 12 | 1.00 | 62 | 9 | 6.9 |
| 10 | 22 | 40 | - | - | 21 | 5 | - | 12 | 1.12 | 62 | 10 | 6.2 |
| 11 | - | - | - | 55 | 8 | - | 25 | 12 | 1.26 | 71 | 17 | 4.2 |
| 12 | - | - | - | 55 | 21 | 12 | - | 12 | 0.84 | 70 | 12 | 5.8 |

Beispiel 13:

Die Arbeitsweise des Beispiels 2 wird wiederholt, es werden jedoch anstelle von Methylmethacrylat 27,5 % Isobornylmethacrylat verwendet.
Man erhält farblose Perlen mit einem mittleren Durchmesser von 0,97 mm und einen Quellgrad in Ethanol von 58 %, in Wasser von 8% und einem Quellverhältnis vom 7,2.

Beispiele 14 und 15:

Gemäß der Arbeitsweise des Beispiels 1, werden Perlen, die 21% HEMA, 12% MAC-Vernetzungsmittel und 67% der folgenden Comonomere enthalten, hergestellt:

| Beispiel | Hydrophobe Comonomere (Gew.-%) | Durchschnittl. Perlengröße (mm) | % Quellung in Ethanol | % Quellung in Wasser | Quellverhältnis (% Ethanol / % Wasser) |
|---|---|---|---|---|---|
| 14 | Cyclohexylmethacrylat (67) | 1,20 | 52 | 8,7 | 6,0 |
| 15 | n-Butylacrylat (67) | 1,00 | 70 | 9,9 | 7,1 |

Die folgenden Beispiele zeigen, daß die Polymere, die mit höheren Alkyl-($C_{4-10}$)-acrylaten und -methacrylaten erhalten wurden, bessere Ethanolquelleigenschaften haben (größeres Verhältnis von Prozent Ethanol zu Prozent Wasser).

Beispiel 16

Gebäß der Arbeitsweise des Beispiels 1 werden Copolymerperlen synthetisiert, und zwar durch Polymerisieren eines Gemisches von 42 g HEMA, 66 g MMA, 66 g EHA, 24 g NVP und 2 g Ethylendimethacrylat (EGDM) anstelle des makromeren Vernetzungs mittels MAC. Man erhält einheitliche kugelförmige Perlen mit einem mittleren Durchmesser von 0,72 mm.

Ihre Zusammensetzung ist:

| | | |
|---|---|---|
| HEMA | : | 21 % |
| NVP | : | 12 % |
| MMA | : | 33 % |
| EHA | : | 33 % |
| EGDM | : | 1 % |

Die Quellung in Ethanol beträgt 60 %, in Wasser 11 % und das Quellverhältnis (% Ethanol / % Wasser) ist = 5,5.

Beispiel 17: Herstellung von Polymerperlen mit expandierter Gelstruktur durch Polymerisation in Gegenwart von inerten Verdünnungsmitteln

Ein 1 000 ml fassender Kunststoffkolben mit glatten Wänden wird ausgerüstet mit einem Rückflußkühler, einem Stickstoffeinlaßstutzen, einem mit einem Temperaturregler verbundenen Thermometer, einem Leitblech und einem Rührer des Ankertyps, der von einem Motor mit variabler Geschwindigkeit angetrieben

wird. Ein langsamer Stickstoffstrom wird während der ganzen Zeit durch den Reaktionskolben geleitet.

In den Kolben werden 360 g einer 20-proz. (Gewichtsbasis) wässrigen Natriumchloridlösung, dann 12,5 g (0,0615 Mol) Magnesiumchlorid-Hexahydrat eingespeist. Die Lösung wird unter schnellem Rühren langsam auf 80 °C erhitzt. Diese Lösung wird dann tropfenweise mit 123 ml (0,123 Mol) einer 1 n Natriumhydroxidlösung versetzt; es bildet sich ein feiner gelatinöser Magnesiumhydroxidniederschlag im Reaktionskolben.

Nachdem die Gesamtmenge an Natriumhydroxid zugegeben worden ist, wird die Rührgeschwindigkeit auf 100 Upm vermindert, und es wird ein Gemisch von 42 g HEMA, 55 g MMA, 55 g EHA, 24 g NVP, 50 g Poly(oxypropylen)-glycol mit einem Molekulargewicht von 3600 und 24 g Makromeres (b) zugegeben, das gelöst 0,2 g tert.-Butylperoctoat als Initiator für die Polymerisation mit freien Radikalen enthält. (Das Makromere (b) wird gemäß Beispiel 1 hergestellt).

Das Reaktionsgemisch wird unter Stickstoff bei 100 Upm und 75 °C 3 h gerührt. Die Temperatur wird dann 1 h auf 100 °C angehoben; dann wird der Kolben auf Raumtemperatur abgekühlt. Es werden 10 ml konzentrierte Salzsäure zugegeben, um das Magnesiumhydroxid-Suspensionsmittel zu lösen. Das Reaktionsgemisch wird durch Käseleinen filtriert. Die isolierten Produktperlen werden mit 2000 ml Wasser gewaschen und über Nacht in 500 ml Ethanol eingeweicht, um eventuell zurückgebliebenes Monomeres zu extrahieren, und dann in einem Soxhlet-Extraktor mit Ethanol extrahiert. Die Perlen werden dadurch isoliert, daß sie durch einen Polyestertuch-Beutel filtriert werden, der zugenäht und in einem Haushalt-Wäschetrockner getrocknet wird. Man erhält einheitliche kugelförmige Perlen in einer Ausbeute von 177 g (89 %) mit einem mittleren Durchmesser von 0,74 mm und einem Quellvermögen in Wasser von 12 % und in Ethanol von 58 %.

Beispiele 18 bis 27:

Gemäß der Arbeitsweise des Beispiels 17 werden Perlen mit einer expandierten Celstruktur hergestellt, und zwar unter Verwendung, der unten angegebenen inerter Verdünnungsmittel. Alle Polymere enthalten 21 % HEMA, 12 % NVP und 12 % NAC-Vernetzungsmittel.

| Bei-spiel | Hydrophobe Comonomere (aa) % MMA | BA | EHA | Verdünnungsmittel | Perlengröße % | mm | % Quellung in Ethanol | Wasser | Quellverhältnis (% Ethanol/ % Wasser) |
|---|---|---|---|---|---|---|---|---|---|
| 17 | 27,5 | - | 27,5 | Poly-(propylenoxid)-glycol MW 3600 | 20 | 0,74 | 58 | 12 | 4,83 |
| 18 | 27,5 | - | 27,5 | " " | 40 | 1,10 | 72 | 9 | 8,00 |
| 19 | 27,5 | 27,5 | - | " " | 20 | 1,10 | 58 | 11 | 5,27 |
| 20 | 27,5 | - | 27,5 | Poly-(ethylenoxid)-glycol MW 4000 | 20 | 0,83 | 64 | 15 | 4,23 |
| 21 | 27,5 | - | 27,5 | " " | 50 | 1,25 | 71 | 10 | 7,10 |
| 22 | 27,5 | - | 27,5 | Dodecanol | 50 | 0,88 | 73 | 10 | 7,30 |
| 23 | 27,5 | - | 27,5 | Poly-(tetramethylenoxid)-glycol MW 2000 | 40 | 1,05 | 69 | 10 | 6,9 |
| 24 | 27,5 | - | 27,5 | " " | 20 | 1,10 | 71 | 12 | 5,9 |
| 25 | 27,5 | - | 27,5 | PLURONIC-L122 (5000) | 20 | 0,95 | 66 | 10 | 6,6 |
| 26 | 27,5 | - | 27,5 | Butylcarbitolacetat | 20 | - | 65 | 10 | 6,5 |
| 27 | 27,5 | - | 27,5 | Dodecanol (75 %) + Poly-(propylenglycol) (25 %) | 50 | 0,96 | 83 | 6 | 13,6 |

Die folgenden Beispiele zeigen die Herstellung und die Wirkstoffabgabe von oralen Wirkstoffdosisformen durch Tränken von Polymeren mit Wirkstofflösungen.

Beispiele 28 bis 38:

10 g trockene Polymerperlen, die gemäß Beispiel 2 hergestellt wurden und einen mittleren Durchmesser von 1,1 $\pm$ 0,1 mm (-16 + 18 Mesh) haben, werden 12 h in eine Lösung von 50 G Oxprenolol•HCl in 50 g Methanol eingetaucht und geschüttelt. Die Perlen werden abfiltriert; der an der Oberfläche haftende Wirkstoff wird mit absolutem Ethanol abgespült und die Perlen werden in Vakuum bei 50 °C bis zum konstanten Gewicht getrocknet (10 h). Der Wirkstoffgehalt der Perlen wird gravimetrisch bestimmt und beträgt 37,3 % Oxprenolol•HCl.

5 g der beladenen Perlen werden in 1 l destilliertem Wasser bei 37,5 °C gerührt. Das Wasser wird durch eine UV-Spektrophotometerzelle geleitet, wo die Geschwindigkeit der Wirkstoffabgabe gemessen wird. 50 % des Wirkstoffs werden innerhalb 35 min und 90 % innerhalb 120 min abgegeben.

Nach der Gleichen Verfahrensweise werden die folgenden Polymer-Wirkstoffzusammensetzunen hergestellt und deren Abgabegeschwindigkeiten Gemessen.

| Beispiel | Polymeres des Beispiels | Polymeres Gew.-Teile | Wirkstoff | % im Lösungsmittel | Lösung Gew.-Teile | Wirkstoffgehalt des Polymeren nach Beladung (Gew.-%) |
|---|---|---|---|---|---|---|
| 28 | 2 | 1 | Oxprenolol•HCl | 50 in Methanol | 10 | 37,3 |
| 29 | 8 | 1 | " " | 60 " " | 1,5 | 44,2 |
| 30 | 9 | 1 | " " | 50 " " | 25 | 38,3 |
| 31 | 9 | 1 | " " | 50 " " | 1 | 30,6 |
| 32 | 2 | 1 | Diclofenac-Natrium | 35 " " | 1,5 | 30,8 |
| 33 | 2 | 1 | " " | 35 " " | 1 | 23,5 |
| 34 | 9 | 1 | " " | 35 " " | 1,5 | 39,4 |
| 35 | 10 | 1 | " " | 35 " " | 1,5 | 31,5 |
| 36 | 2 | 1 | Baclofen | 30 in 75/25 Ethanol/$H_2O$ | 1,25 | 21,0 |
| 37 | 9 | 1 | " | 30 " " | 1,05 | 17,9 |
| 38 | 11 | 1 | Oxprenolol•HCl | 50 in Methanol | 2,0 | 51,3 |

In keinem Fall werden 50 % des aufgebrachten Wirkstoffs vor 30 min nach der Behandlung mit destilliertem Wasser bei 37,5 °C abgegeben.

Beispiele 39 - 50: Gemäß der Arbeitsweise des Beispiels 28 und unter den unten beschriebenen Bedingungen werden die folgenden Polymer-Wirkstoff-Zusammensetzungen als Perlen in Gegenwart von inerten Verdünnungsmitteln hergestellt.

| Beispiel | Polymeres des Beispiels | Verdünnungsmittel * | Maschengröße der Perlen | Polymeres Gew.-Teile | Beladungsbedingungen: Wirkstoff | Beladungsbedingungen: % Lösungsmittel | Lösung Gew.-Teile | Wirkstoffgehalt des Polymeren nach Beladen (Gew.-%) |
|---|---|---|---|---|---|---|---|---|
| 39 | 18 | PPO | 18 | 1 | Oxprenolol·HCl | 50 in Methanol | 2 | 50,7 |
| 40 | 18 | PPO | 30 | 1 | Diclofenac-Natrium | 35 in Methanol | 3 | 51,4 |
| 41 | 19 | PPO | 18 | 1 | Oxprenolol·HCl | 50 in Methanol | 2 | 50,8 |
| 42 | 25 | PLUR | 18 | 1 | " " | 50 in Wasser | 2 | 43,7 |
| 43 | 24 | PTMO | 18 | 1 | " " | 50 in Methanol | 2 | 46,9 |
| 44 | 26 | BCAC | 18 | 1 | " " | 50 in Methanol | 5 | 48,1 |
| 45 | 23 | PTMO | 20 | 1 | Carbamazepin | 10 " " | 2 | 16,3 |
| 46 | 22 | Dodecanol | 18 | 1 | Diclofenac-Natrium | 35 " " | 5 | 62,7 |
| 47 | 18 | PPO | 25 | 1 | " " | 35 " " | 3 | 51,2 |
| 48 | 18 | PPO | 40 | 1 | " " | 35 " " | 3 | 49,2 |
| 49 | 18 | PPO | 200 | 1 | Tegritol | 20 " " | 3 | 21,8 |
| 50 | 27 | Dodecanol + PPO | 16 | 1 | Metropolol·HCl | 50 " " | 3 | 45,2 |

* PPO = Poly-(propylenoxid)-glycol Molekulargewicht 3600
PLUR = Pluronic-L122 (5000)
PTMO= Poly-(tetramethylenoxid)-glycol Molekulargewicht 2000
BCAC= Butylcarbitolacetat

**Beispiele 51 - 57:** Gemäß der Arbeitsweise des Beispiels 38 werden Perlen der folgenden Zusammensetzungen hergestellt; ihr Quellgrad in Ethanol und Wasser wird angegeben.

| Beispiel | Zusammensetzung, % | | | | | | | Verdünnungsmittel % | | Perlengröße (mm) | Quellung in Ethanol % | Quellung in Wasser % | Quellverhältnis (% Ethanol/ % Wasser) |
|---|---|---|---|---|---|---|---|---|---|---|---|---|---|
| | NAC | HEMA | NVP | MMA | EHA | GMA | EGDM | DD | PPO | | | | |
| 51 | 12 | 21 | 12 | 7,5 | 27,5 | - | 20 | 40 | | 0,99 | 46,9 | 8,0 | 5,86 |
| 52 | 12 | 21 | 12 | 7,5 | 27,5 | - | 20 | | 40 | 0,95 | 51,6 | 34,8 | 1,48 |
| 53 | 12 | 21 | 12 | 15 | - | 40 | - | 40 | | 0,57 | 48,6 | 19,7 | 2,47 |
| 54 | - | 21 | 12 | 33 | 33 | - | 1 | 40 | | 0,77 | 73,0 | 9,0 | 8,11 |
| 55 | - | 21 | 12 | 33 | 33 | - | 1 | | 40 | 0,96 | 80,2 | 9,0 | 8,91 |
| 56 | - | 10 | - | 49 | - | 40 | 1 | 40 | | 0,88 | 42,8 | 10,8 | 3,96 |
| 57 | - | 10 | - | 10 | - | 40 | 40 | 40 | | 1,12 | 50,1 | 53,6 | 0,934 |

DD = Dodecanol

PPO - Poly-(propylenoxid)-glycol

Von beiden Verdünnungsmitteln ist PPO das schlechtere Lösungsmittel für das Polymere und gibt eine macroporöse Struktur (wie die hohe Quellung in Wasser zeigt) mit weniger Kurzketten-Vernetzungsmittel (EGDM) (Beispiel 52) als DD (Beispiel 51).

Die folgenden beiden Beispiele zeigen die Eignung der erfindungsgemäßen wirkstoffbeladenen Perlen für die Durchführung des Verfahrens der kontrollierten Extraktion, wobei eine viel längere Wirkstoffabgabe bei gleichzeitiger Aufrechterhaltung eines hohen Wirkstoffgehaltes erreicht werden kann.

Beispiel 58:

Zwei 5 g-Proben von wirkstoffbeladenen Perlen des Beispiels 48 werden 10 min (Behandlung A) und 20 min (Behandlung B) in Wasser getaucht und dann schnell gefriergetrocknet.

Die folgende Tabelle zeigt die entstandenen Wirkstoffbeladungen und die Zeiten, bei denen 50 % und 90 % des Wirkstoffes in ein gerührtes Volumen (1 1) Wasser bei 37,5 °C abgegeben werden, wobei das Wasser kontinuierlich durch eine UV-Spektrophotometerzelle geleitet wird.

| Perlen des Beispiels | Wirkstoff | % | Zeit (h) bis zur Abgabe von 50 % | 90 % |
|---|---|---|---|---|
| 48 | Diclofenac-Natrium | 49,2 | 0,3 | 1,65 |
| Nach Behandlung A | | 42,3 | 2,45 | 6,25 |
| Nach Behandlung B | | 37,4 | 5,33 | 9,71 |

Beispiel 59

4 g der wirkstoffbeladenen Perlen des Beispiels 41 werden 10 min in Aceton getaucht und dann schnell in Vakuum gefriergetrocknet. Der Wirkstoffgehalt wird bestimmt und die Wirkstoffabgabegeschwindigkeitin werden wie oben beschrieben gemessen; man erhält folgende Ergebnisse:

| Perlen des Beispiels | Wirkstoff | % | Zeit (h) bis zur Abgabe von 50 % | 90 % |
|---|---|---|---|---|
| 41 | Oxprenolol·HCl | 50,8 | 0,2 | 1,68 |
| nach Behandlung | | 46,3 | 1,12 | 3,27 |

Beispiele 60 - 63:

Die folgenden Beispiele zeigen die Überlegenheit der Perlen, die in Gegenwart eines polymeren inerten Verdünnungsmittels hergestellt worden sind, gegenübor Perlen, die mit einem inerten Verdünnungsmittel mit niedrigem Molekulargewicht hergestellt worden sind, und zwar hinsichtlich des geringeren Verschleppens bei der Wirkstoffabgabe.

5 g-Proben der gemäss den Beispielen 17, 18 und 22 hergestellten Perlen, die gemäß den Beispielen 28 bis 38 mit Oxprenolol• HCl und Diclofenac beladen wurden, werden in einem Verfahren zur kontrollierten Extraktion behandelt. Ihre Wirkstoffabgabe wird dadurch gemessen, daß sie in 1 l destillierter Wasser bei 37,5 °C gerührt werden, wobei das Wasser durch eine UV-Spektrophotometerzelle geleitet wird. Wie aus der Tabelle ersichtlich ist, führt das polymere Verdünnungsmittel Polypropylenoxid (PPO) zu 90 % Abgabe innerhalb 9 h, wogegen das niedrigmolekulare Dodecanol (DD) zu einem übermäßigen Verschleppen führt.

Beispiele 60 - 63:

| Bei-spiel | Perlen Beispiel | Maschen-größe | Verdünnungs-mittel | Wirkstoff und kontrollierte Extraktion | Wirkstoff-beladung % | Zeit bis zur Abgabe von 50 % des aufgebrachten Wirkstoffs (h) | 90 % |
|---|---|---|---|---|---|---|---|
| 60 | 17 | 18 | 20 % PPO | Oxprenolol·HCl 10 min Waschen mit Aceton dann | 31 | 4,4 | 9,1 |
| 61 | 22 | 18 | 50 % DD | Gefriertrocknen | 41 | 2,0 | > 20 (Schwanz-bildung) |
| 62 | 18 | 30 | 40 % PPO | Diclofenac 10 min Waschen mit Wasser, dann | 45 | 3,4 | 8,6 |
| 63 | 22 | 30 | 50 % DD | Gefriertrocknen | 51 | 0,7 | > 20 (Schwanz-bildung) |

## Ansprüche

1. Eine Zusammensetzung mit kontrollierter Wirkstoffabgabe, bestehend aus:
   (A) einem vernetztem Copolymerem, das in Ethanol zu einem gequollenen, mindestens 40 Gew.-% Ethanol enthaltenden Copolymeren und in Wasser zu einem gequollenen, nicht über 20 Gew.-% Wasser enthaltenden Copolymeren quellen kann, wobei das Quellverhältnis [Prozent Ethanol zu Prozent Wasser] 2:l bis 22:1 beträgt und das Copolymere das Copolymerisationsprodukt ist von
   (a) 50 bis 99 Gew.-% des Copolymeren (aa) eines wasserunlöslichen monoolefinischen Monomeren oder eines Gemisches solcher Monomeren oder eines Gemisches dieser Monomeren mit 0 bis 45 Gew.-% der Gesamtmonomere (bb) eines wasserlöslichen monoolefinischen Monomeren oder eines Gemisches solcher wasserlöslicher Monomere, worin mindestens ein Drittel des Gewichtes des wasserunlöslichen Monomeren des Bestandteiles
   (a) ein Monomeres mit einer Alkylgruppe mit 4 bis 21 Kohlenstoffatomen ist, mit
   (b) 50 bis 1 Gew.-% des Copolymeren eines Divinyl- oder Polyvinyl-Vernetzungsmittels mit einem Molekulargewicht von 100 bis 10 000, wobei jedoch (b) nicht über 20 Mol-% des Bestandteils (a) ausmacht, und
   (B) einer wirkungsvollen Menge eines pharmazeutischen Medikaments.

2. Eine Zusammensetzung gemäss Anspruch 1, in der das Copolymere (A) ein Quellverhältnis [Prozent Ethanol zu Prozent Wasser] von 3:1 bis 15:1, bevorzugt von 4:1 bis 8:1, aufweist.

3. Eine Zusammensetzung gemäss Anspruch 1, in der das Copolymere (A) ein Quellverhältnis [Prozent Ethanol zu Prozent Wasser] von 4:1 bis. 22:1 aufweist.

4. Eine Zusammensetzung gemäss Anspruch 1, in der im Copolymeren (A) mindestens die Hälfte des Gewichtes des wasserunlöslichen Monomeren des Bestandteiles (a) ein Monomeres mit einer Alkylgruppe mit 4 bis 21 Kohlenstoffatomen ist.

5. Eine Zusammensetzung nach Anspruch 1, in der das Copolymere (A) das Copolymerisationsprodukt von 75 bis 98 Gew.-% des Bestandteils (a) und 25 bis 2 Gew.-% des Bestandteils (b) ist.

6. Eine Zusammensetzung nach Anspruch 1, in der der Bestandteil (a) aus 70 bis 95 Gew.-% des wasserunlöslichen Monomeren (aa) und 30 bis 5 Gew.-% des wasserlöslichen Monomeren (bb) besteht.

7. Eine Zusammensetzung nach Anspruch 1, in der das wasserunlösliche Monomere des Bestandteiles (a) Methylmethacrylat, n-Butylacrylat, n-Butylmethacrylat, 2-Ethylhexylacrylat, n-Octylacrylat, n-0ctylmethacrylat, Glycidylmethacrylat, Vinylacetat oder eines ihrer Gemische ist.

8. Eine Zusammensetzung nach Anspruch 1, in der 10 bis 60 Gew.-% des Bestandteils (a) n-Butylacrylat oder 2-Ethylhexylacrylat sind.

9. Eine Zusammensetzung nach Anspruch 1, in der das wasserlösliche Monomere des Bestandteils (a) N-Vinyl-2-pyrrolidon, 2-Hydroxyethylacrylat, 2-Hydroxyethylmethacrylat, Acrylsäure, Methacrylsäure, N,N-Dimethylacrylamid, 2-Dimethylaminoethylmethacrylat oder eines ihrer Gemische ist.

10. Eine Zusammensetzung nach Anspruch 1, in der das Vernetzungsmittel des Bestandteils (b) ein hydrophohes Macromeres mit endständigen diolefinischen Gruppen und einem Molekulargewicht von etwa 400 bis etwa 8000 ist, wobei das Macromere die Formel

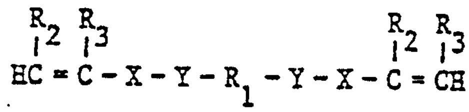

hat, in der $R_1$ eine polykondensierte Kette mit einem Molekulargewicht von etwa 200 bis 8000 ist und der Pest eines Poly-(propylenoxid)- oder Poly(tetramethylenoxid)-glycols mit Etherbindungen ist;
$R_2$ ein Wasserstoff, eine Methylgruppe oder - $CH_2COOR_4$ ist, wobei $R_4$ ein Wasserstoffatom oder eine Alkylgruppe mit bis zu 10 Kohlenstoffatomen ist,
$R_3$ ein Wasserstoffatom oder $COOR_4$ ist, mit der Maßgabe, daß mindestens einer der Feste $R_2$ und $R_3$ ein Wasserstoffatom ist, X Oxa, -COO-, oder - $CONR_5$- ist, wobei $R_5$ ein Wasserstoffatom oder eine Alkylgruppe mit bis zu 5 Kohlenstoffatomen ist, und
Y eine direkte Bindung oder der Rest $-R_6-Z_1-CO-NH-R_7-NH-CO-Z_2-$ist, wobei $R_6$ an X gebunden ist und eine geradkettige oder verzweigte Alkylengruppe mit bis zu 7 Kohlenstoffatomen darstellt, $Z_1$ und $Z_2$ Oxa oder $NR_5$ sind und $R_7$ der zweiwertige Rest eines aliphatischen oder aromatischen Diisccyanats ist, mit der Maßgabe, daß wenn X Oxa ist, Y keine direkte Bindung ist und $R_2$ und $R_3$ Wasserstoffatome sind.

**11.** Eine Zusammensetzung nach Anspruch 10, in der $R_1$ eine Polypropylenoxid- oder Polytetramethylenoxidkette mit einem Molekulargewicht von etwa 600 bis etwa 4000 ist.

**12.** Eine Zusammensetzung nach Anspruch 10, in der das Makromere ein Polytetramethylenoxidglycol mit einem Molekulargewicht von etwa 600 bis etwa 4000 ist, das Toluol- oder Isophoron-diisocyanat-Endgruppen aufweist, die mit 2 Mol eines Hydroxyalkylacrylats oder -methacrylats umgesetzt worden sind, wobei dessen Alkylgruppe 2 bis 4 Kohlenstoffatome hat.

**13.** Eine Zusammensetzung nach Anspruch 1, in der das Vernetzungsmittel (b) ein $C_{2`6}$-Alkylendiacrylat, $c_{2\text{-}6}$-Alkylendimethacrylat, 3-Oxapentamethylendiacrylat, 3-Oxapentamethylendimethacrylat, Trimethylolpropantriacrylat oder eines ihrer Gemische ist.

**Claims**

**1.** A composition with controlled active ingredient release consisting of:
(A) a crosslinked copolymer, capable of swelling in ethanol to give a swollen copolymer containing at least 40 % by weight of ethanol, and capable of swelling in water to given a swollen copolymer containing no more than 20 % by weight of water, wherein the swelling ratio [% ethanol : % water] is 2:1 to 22:1 and the copolymer is the copolymerisation product of:
(a) 50 to 99 % by weight of the copolymer of (aa) a water-insoluble monoolefinic monomer, or a mixture of such monomers, or a mixture of such monomers with 0 to 45 % by weight of total monomers of (bb) a water-soluble monoolefinic monomer, or a mixture of such water-soluble monomers, wherein at least a third of the weight of the water-insoluble monomer of component (a) is a monomer containing an alkyl group having from 4 to 21 carbon atoms, with
(b) 50 to 1 % by weight of the copolymer of a divinyl or polyvinyl crosslinking agent having a molecular weight of 100 to 10,000, but where (b) is not more than 20 mol % of component (a), and
(B) an effective amount of a pharmaceutical medicament.

**2.** A composition according to claim 1, wherein the copolymer (A) exhibits a swelling ratio [% ethanol : % water] of 3:1 to 15:1, preferably 4:1 to 8:1.

**3.** A composition according to claim 1, wherein the copolymer (A) exhibits a swelling ratio [% ethanol : % water] of 4:1 to 22:1.

**4.** A composition according to claim 1, wherein in the copolymer (A) at least half the weight of the water-insoluble monomer of component (a) is a monomer containing an alkyl group having from 4 to 21 carbon atoms.

**5.** A composition according to claim 1, wherein the copolymer (A) is the copolymerisation product of 75 to

98 % by weight of component (a) and 25 to 2 % by weight of component (b).

6. A composition according to claim 1, wherein component (a) consists of 70 to 95 % by weight of water-insoluble monomer (aa) and 30 to 5 % by weight of water-soluble monomer (bb).

7. A composition according to claim 1, wherein the water-insoluble monomer of component (a) is methyl methacrylate, n-butyl acrylate, n-butyl methacrylate, 2-ethylhexyl acrylate, n-octyl acrylate, n-octyl methacrylate, glycidyl methacrylate, vinyl acetate or a mixture thereof.

8. A composition according to claim 1, wherein 10 to 60 % by weight of component (a) is n-butyl acrylate or 2-ethylhexyl acrylate.

9. A composition according to claim 1, wherein the water-soluble monomer of component (a) is N-vinyl-2-pyrrolidone, 2-hydroxyethyl acrylate, 2-hydroxyethyl methacrylate, acrylic acid, methacrylic acid, N,N-dimethylacrylamide, 2-dimethylaminoethyl methacrylate or a mixture thereof.

10. A composition according to claim 1, wherein the crosslinking agent of component (b) is a hydrophobic macromer having terminal diolefinic groups and a molecular weight of from about 400 to about 8000, the macromer having the formula

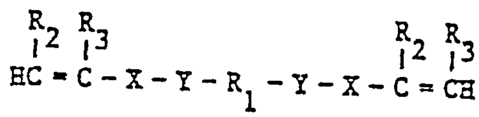

wherein $R_1$ is a polycondensate chain having a molecular weight of from about 200 to 8000 and is the residue of a poly(propylene oxide) or poly(tetramethylene oxide) glycol having ether linkages;
$R_2$ is hydrogen, a methyl group or - $CH_2COOR_4$, wherein $R_4$ is a hydrogen atom or an alkyl group having up to 10 carbon atoms,
$R_3$ is a hydrogen atom or $COOR_4$, with the proviso that at least one of the radicals $R_2$ and $R_3$ is a hydrogen atom; X is oxa, -COO-, or - $CONR_5$- wherein $R_5$ is a hydrogen atom or an alkyl group having up to 5 carbon atoms and Y is a direct bond or the radical
$-R_6-Z_1-CO-NH-R_7-NH-CO-Z_2-$, wherein $R_6$ is linked to X and represents a straight-chain or branched alkylene group having up to 7 carbon atoms; $Z_1$ and $Z_2$ are oxa or $NR_5$, and $R_7$ is the divalent radical of an aliphatic or aromatic diisocyanate, with the proviso that when X is oxa, Y is not a direct bond and $R_2$ and $R_3$ are hydrogen atoms.

11. A composition according to claim 10, wherein $R_1$ is a polypropylene oxide or polytetramethylene oxide chain having a molecular weight of from about 600 to about 4000.

12. A composition according to claim 10, wherein the macromer is a polytetramethylene oxide glycol having a molecular weight of from about 600 to about 4000, that contains toluene or isophorone diisocyanate terminal groups that have been reacted with 2 moles of a hydroxy alkyl acrylate or methacrylate, wherein alkyl has from 2 to 4 carbon atoms.

13. A composition according to claim 1, wherein the crosslinking agent (b) is a $C_{2-6}$alkylene diacrylate, a $C_{2-6}$alkylene dimethacrylate, 3-oxapentamethylene diacrylate, 3-oxapentamethylene dimethacrylate, trimethylolpropane triacrylate or a mixture thereof.

## Revendications

1. Une composition a libération réglée de matières actives, qui comprend :
(A) un copolymère réticulé pouvant gonfler dans de l'éthanol en formant un copolymère gonflé qui contient au moins 40% en poids d'éthanol et dans de l'eau en formant un copolymère gonflé ne contenant pas plus de 20% en poids d'eau, le rapport en pourcentages du gonflement dans l'éthanol par rapport au gonflement dans l'eau étant compris entre 2:1 et 22:1 et ce copolymère étant un produit

de copolymérisation de (a) 50 à 99% du poids du copolymère, (aa) d'un monomère mono-oléfinique insoluble dans l'eau ou de plusieurs de tels monomères ou encore de ces monomères avec de 0 à 45%, du poids de l'ensemble des monomères, (bb) d'un monomère monooléfinique hydrosoluble ou de plusieurs de tels monomères hydrosolubles, un tiers au moins en poids du monomère insoluble dans l'eau du constituant (a) étant un monomère avec un alkyle en $c_4$ à $c_{21}$, avec

(b) 50 à 1% du poids du copolymère, d'un agent réticulant divinylique ou polyvinylique ayant une masse moléculaire de 100 à 10 000, mais le constituant (b) ne représentant pas plus de 20 mol% du constituant (a), et

(B) une proportion efficace d'un médicament.

**2.** Une composition selon la revendication 1 dont le copolymère (A) donne un rapport des gonflements (% dans l'éthanol au % dans l'eau) compris entre 3:1 et 15:1, de préférence entre 4:1 et 8:1.

**3.** Une composition selon la revendication 1 dont le copolymère (A) donne un rapport des gonflements dans l'éthanol et l'eau compris entre 4:1 et 22:1.

**4.** Une composition suivant la revendication 1 dans laquelle dans le copolymère (A) au moins la moitié du poids des monomères insolubles dans l'eau du constituant (a) est un monomère avec un alkyle ayant de 4 à 21 atomes de carbone.

**5.** Une composition selon la revendication 1 dont le copolymère (A) est le produit de copolymérisation de 75 à 98% en poids du constituant (a) avec de 25 à 2% du constituant (b).

**6.** Une composition selon la revendication 1 dans laquelle le constituant (A) est formé de 70 à 95% en poids du monomère (aa) insoluble dans l'eau et de 30 à 5% du monomère hydrosoluble (bb).

**7.** Une composition selon la revendication 1 dans laquelle le monomère insoluble dans l'eau du constituant (a) est le méthacrylate de méthyle, l'acrylate de n-butyle, le méthacrylate de n-butyle, l'acrylate de 2-éthylhexyle, l'acrylate de n-octyle, le méthacrylate de n-octyle, le méthacrylate de glycidyle, l'acétate de vinyle ou un mélange de plusieurs de ces composés.

**8.** Une composition selon la revendication 1 dans laquelle de 10 à 60% en poids du constituant (a) sont l'acrylate de n-butyle ou l'acrylate de 2-éthylhexyle.

**9.** Une composition selon la revendication 1 dans laquelle le monomère hydrosoluble du constituant (a) est la N-vinyl-2-pyrrolidone, l'acrylate de 2-hydroxyéthyle, le méthacrylate de 2-hydroxyéthyle, l'acide acrylique ou méthacrylique, le N,N-diméthylacrylamide, le méthacrylate de 2-diméthylaminoéthyle ou un mélange de plusieurs de ces composés.

**10.** Une composition selon la revendication 1 dans laquelle l'agent réticulant du constituant (b) est un macromère hydrophobe à groupes dioléfiniques terminaux ayant une masse moléculaire d'environ 400 à 8000, de formule :

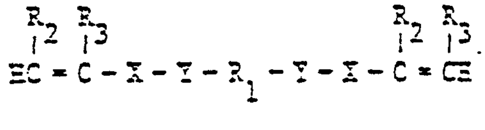

dans laquelle

$R_1$ est une chaîne polycondensée de masse moléculaire environ 200 à 8000 et le radical d'un poly-(oxypropylène)-ou poly-(oxytétraméthylène)-glycol à liaisons éther,

$R_2$ un atome d'hydrogène, un groupe méthyle ou - $CH_2COOR_4$, $R_4$ étant un atome d'hydrogène ou un alkyle pouvant avoir jusqu'à 10 atomes de carbone,

$R_3$ est un atome d'hydrogene ou un groupe $COOR_4$ avec la condition qu'au moins l'un des radicaux $R_2$ et $R_3$ soit un atome d'hydrogène,

X le groupe oxa, -COO- ou - $CONR_5$, $R_5$ étant un atome d'hydrogène ou un alkyle pouvant avoir

jusqu'à 5 atomes de carbone, et

Y représente une liaison directe ou un radical $-R_6-Z_1-CO-NH-R_7-NH-CO-Z_2-$, $R_6$ étant lié à X et étant un alkylène ramifié ou linéaire pouvant avoir jusqu'à 7 atomes de carbone, $Z_1$ et $Z_2$ étant chacun un groupe oxa ou $NR_5$, et $R_7$ un radical diisocyanate divalent aliphatique ou aromatique, avec la condition que si X est le groupe oxa, Y ne soit pas une liaison directe et $R_2$ et $R_3$ soient des atomes d'hydrogène.

**11.** Une composition selon la revendication 10 dans laquelle $R_1$ est une chaîne de polyoxypropylène ou de polyoxytétraméthylène ayant une masse moléculaire d'environ 600 à 4000.

**12.** Une composition selon la revendication 10 dans laquelle le macromère est un polyoxytétraméthylèneglycol de masse moléculaire environ 600 à 4000, à groupes terminaux de diisocyanate de toluène ou d'isophorone, ayant réagi avec 2 moles d'un acrylate ou méthacrylate d'hydroxyalkyle dont l'alkyle a de 2 à 4 atomes de carbone.

**13.** Une composition selon la revendication 1 dans laquelle l'agent réticulant (b) est un diacrylate ou un diméthacrylate d'alkylène en $C_{2-6}$, le diacrylate ou le diméthacrylate de 3-oxapentaméthylène, le triacrylate de triméthylol-propane ou un mélange de plusieurs de ces composés.